# EUROPEAN PATENT APPLICATION

(11) **EP 4 394 044 A1**
(43) Date of publication of application: **03.07.2024**
(21) Application number: 22869861.9
(22) Date of filing: 06.09.2022
(51) Int. Cl.: C12Q 1/04, A61P 15/00, A61P 31/04

(54) **METHOD FOR DETECTING BACTERIA OF GENUS FUSOBACTERIUM IN ORDER TO DIAGNOSE ENDOMETRIOSIS**

(30) Priority: 17.09.2021 JP 2021152197
(71) Applicant: National University Corporation Tokai National Higher Education and Research System, Nagoya-shi, Aichi 464-8601 (JP); EIKEN KAGAKU KABUSHIKI KAISHA, Tokyo 110-8408 (JP)
(72) Inventor: MURAOKA Ayako, Nagoya-shi, Aichi 464-8601 (JP); KONDO Yutaka, Nagoya-shi, Aichi 464-8601 (JP); KAKU Toshisuke, Shimotsuga-gun, Tochigi 329-0114 (JP); MATSUI Atsuka, Shimotsuga-gun, Tochigi 329-0114 (JP)
(74) Representative: Boult Wade Tennant LLP
(86) International application number: PCT/JP2022/033474
(87) International publication number: WO 2023/042714

(57) **Abstract**

A method for detecting a *Fusobacterium* bacterium for diagnosing endometriosis according to one aspect of the present invention includes a step of detecting a *Fusobacterium* bacterium in a sample collected from a subject, wherein the detection of the presence of the *Fusobacterium* bacterium in the sample indicates that the subject is likely suffering from or likely to suffer from endometriosis. According to the present invention, a simple diagnosis of endometriosis becomes possible by detecting the causative bacterium of endometriosis.

## Description

### Technical Field

The present invention relates to a method for detecting a *Fusobacterium* bacterium for diagnosing endometriosis, a therapeutic agent for endometriosis, and a method for treating endometriosis.

### Background Art

Endometriosis is a disease in which endometrial tissue grows in parts of the body other than the uterus (such as ovaries and peritoneum). Endometriosis is a disease that causes chronic pelvic pain, infertility, canceration, and the like, and significantly impairs a woman's quality of life (QOL) throughout the life, but the details of its cause are unknown and there is no fundamental cure for the disease. Pseudomenopause therapy by hormone therapy is used as symptomatic treatment, but it has side effects and cannot be applied to women who wish to become pregnant. A treatment which involves removing a lesion by surgery is also performed, but there may be a high postoperative recurrence rate.

Regarding the cause of endometriosis, Non-Patent Literature 1 and 2 suggest a relationship between gram-negative bacteria and endometriosis, but a specific causative bacterium have not been identified.

### Citation List

### Non-Patent Literature

Non-Patent Literature 1: Human Reproduction, Vol. 17, No. 7, pp. 1704-1708, 2002
Non-Patent Literature 2: Journal of Japan Society of Endometriosis, 33, pp. 62-67, 2012

### Summary of Invention

### Technical Problem

An object of the present invention is to enable diagnosis of endometriosis by detecting a causative bacterium of endometriosis.

### Solution to Problem

As a result of extensive studies, the present inventors have found that the infection of the endometrium with a *Fusobacterium* bacterium is involved in the onset of endometriosis, thus leading to the realization of the present invention.

The present invention has the following aspects.
[1] A method for detecting a *Fusobacterium* bacterium for diagnosing endometriosis including: detecting a *Fusobacterium* bacterium in a sample collected from a subject, wherein the detection of the presence of the *Fusobacterium* bacterium in the sample indicates that the subject is likely suffering from or likely to suffer from endometriosis. The *Fusobacterium* bacterium may be *Fusobacterium nucleatum.* The sample may be blood, plasma, serum, urine, endometrial cells, vaginal swab fluid, or menstrual blood.
[2] A therapeutic agent for endometriosis containing an antibacterial agent against a *Fusobacterium* bacterium as an active component.
[3] A method for treating endometriosis, including: a step of administering an antibacterial agent to a subject whose endometrium is infected with a *Fusobacterium* bacterium. A step of detecting the *Fusobacterium* bacterium in a sample collected from the subject to confirm whether or not the endometrium of the subject is infected with the *Fusobacterium* bacterium may be performed before the step of administering an antibacterial agent. The antibacterial agent may be administered vaginally.
   The present invention further has the following aspects.
[4] A method for collecting data for diagnosing endometriosis, including: detecting a *Fusobacterium* bacterium in a sample collected from a subject.
[5] A use of an antibacterial agent for the manufacture of a medicament for the treatment of endometriosis caused by infection with a *Fusobacterium* bacterium.

### Advantageous Effects of Invention

According to the present invention, a simple diagnosis of endometriosis becomes possible by detecting the causative bacterium of endometriosis. Since bacteria can be detected even in trace amounts, it can also be expected to detect early endometriosis that cannot be captured by diagnostic imaging. In addition, according to the present invention, endometriosis can be treated by sterilizing the causative bacterium of endometriosis or suppressing proliferation thereof. Such treatment can also be expected to be effective in preventing recurrence of endometriosis.

### Brief Description of Drawings

FIG. 1 shows proportions of 16S rRNA of *Fusobacterium* within the total 16S rRNA in the normal human endometrial tissues and the endometrial tissues of the patients with endometriosis.
FIG. 2 shows FISH images of the normal human endometrial tissue and the endometrial tissue and ovarian tissue from a patient with endometriosis in which EUB338, FUSO, and FUS664 are used as probes.
FIG. 3(A) shows the endometriotic lesions in mice inoculated intraperitoneally with endometria infected with the bacteria. FIG. 3(B) shows the number of the lesions, and FIG. 3(C) shows the total weight of the lesions.
FIG. 4 shows FISH images of the endometriotic lesions in mice inoculated intraperitoneally with endometria infected with the bacteria in which EUB338 and FUSO are used as probes.
FIG. 5(A) shows the endometriotic lesions in mice inoculated intraperitoneally with the endometrial tissues of the donor mice treated with the antibacterial agent. FIG. 5(B) shows the number of the lesions, and FIG. 5(C) shows the total weight of the lesions.
FIG. 6 shows FISH images of the endometriotic lesions in mice inoculated intraperitoneally with the endometrial tissues of the donor mice treated with the antibacterial agent in which EUB338 and FUSO are used as probes.
FIG. 7(A) shows the endometriotic lesions in the endometriosis mice treated with the antibacterial agent. FIG. 7(B) shows the number of the lesions, and FIG. 7(C) shows the total weight of the lesions.

### Description of Embodiment

A method for detecting a *Fusobacterium* bacterium for diagnosing endometriosis according to one aspect of the present invention includes a step of detecting a *Fusobacterium* bacterium in a sample collected from a subject. If the presence of the *Fusobacterium* bacterium is detected in the sample collected from a subject, it is likely that the endometrium of the subject is infected with the *Fusobacterium* bacterium. Therefore, the detection of the presence of the *Fusobacterium* bacterium in the sample indicates that the subject is likely suffering from or likely to suffer from endometriosis. This result can be used by a medical doctor as an indicator for diagnosing endometriosis.

The *Fusobacterium* bacterium is not particularly limited, and may be, for example, *Fusobacterium nucleatum.*

The subject is not particularly limited as long as it has a uterus. The subject may be, for example, a human subject, and may be a human subject who may suffer from endometriosis or who is suspected of suffering from endometriosis.

The sample is not particularly limited as long as it can detect the *Fusobacterium* bacterium that have infected the endometrium, and may be, for example, blood, plasma, serum, urine, endometrial cells, vaginal swab fluid, menstrual blood, cervical cells, or ovarian tissue. From the viewpoint of collecting the sample without or with minimal invasion, the sample is preferably blood, plasma, serum, urine, endometrial cells, vaginal swab fluid, or menstrual blood. From the viewpoint of specifically detecting endometrial infection, the sample is more preferably endometrial cells, vaginal swab fluid, or menstrual blood.

The method for detecting a *Fusobacterium* bacterium in a sample is not particularly limited, and examples thereof include conventionally known bacterial detection methods such as genetic testing by nucleic acid amplification methods such as a PCR (polymerase chain reaction) method and a LAMP (loop-mediated isothermal amplification) method in which primers specific to a *Fusobacterium* bacterium are used.

For example, the step of detecting a *Fusobacterium* bacterium in a sample collected from a subject may include a step of performing a nucleic acid amplification reaction on DNA derived from the sample using primers specific to the *Fusobacterium* bacterium (nucleic acid amplification step), and may further include a step of detecting an amplification product of the nucleic acid amplification reaction. The detection of an amplification product may be performed after the nucleic acid amplification step or may be performed in real time during the nucleic acid amplification step. If the presence of an amplification product is detected, it can be said that there is a *Fusobacterium* bacterium in the sample.

DNA derived from a sample may be DNA extracted and/or isolated from a sample by known techniques. Primers specific to a *Fusobacterium* bacterium mean one or more kinds of primers capable of amplifying a DNA region specific to the *Fusobacterium* bacterium.

The method for detecting an amplification product is not particularly limited, and known technology may be used. An amplification product may be detected, for example, by using a labeling probe such as a fluorescence labeling probe or a fluorescent intercalator, or by performing electrophoresis on a reaction solution. Alternatively, in a case where magnesium ions are contained in a reaction solution of the nucleic acid amplification reaction, the amplification product may be detected by measuring the turbidity of the reaction solution.

According to the method according to the above-described aspect of the present invention, it is possible to obtain data that assists diagnosis of endometriosis. Accordingly, it can be said that one aspect of the present invention is a method for collecting data for diagnosing endometriosis, the method including a step of detecting a *Fusobacterium* bacterium in a sample collected from a subject. The details of the step of detecting a *Fusobacterium* bacterium in a sample collected from a subject are as described above. Data of detection results of the *Fusobacterium* bacterium in the sample can be used by a medical doctor for diagnosing endometriosis.

The method for collecting data for diagnosing endometriosis may further include a step of determining the likelihood that the subject is suffering from or may suffer from endometriosis based on the detection results of the *Fusobacterium* bacterium (the presence or absence of the *Fusobacterium* bacterium). In this step, when the presence of the *Fusobacterium* bacterium is detected in the sample, it is determined that the subject is likely suffering from or likely to suffer from endometriosis. The determination data obtained in this manner can be used by a medical doctor for diagnosing endometriosis.

A therapeutic agent for endometriosis according to one aspect of the present invention contains an antibacterial agent against a *Fusobacterium* bacterium as an active component. The therapeutic agent for endometriosis may specifically be a therapeutic agent for use in the treatment of endometriosis caused by infection with a *Fusobacterium* bacterium. In addition, another aspect of the present invention is a use of an antibacterial agent for the manufacture of a medicament for the treatment of endometriosis caused by infection with a *Fusobacterium* bacterium.

The details of the *Fusobacterium* bacterium are as described above. The antibacterial agent is not particularly limited as long as it is an antibacterial agent that can sterilize the *Fusobacterium* bacterium or suppress its proliferation, and examples thereof include metronidazole antibacterial agents and chloramphenicol antibacterial agents.

A method for treating endometriosis according to one aspect of the present invention includes a step of administering an antibacterial agent to a subject whose endometrium is infected with a *Fusobacterium* bacterium.

As described above, whether or not the endometrium of a subject is infected with a *Fusobacterium* bacterium can be confirmed based on whether or not the *Fusobacterium* bacterium is detected in a sample collected from the subject. Namely, in one embodiment, the method for treating endometriosis may include a step of detecting a *Fusobacterium* bacterium in a sample collected from a subject and a step of administering an antibacterial agent to the subject when the presence of the *Fusobacterium* bacterium is detected in the sample.

The details of the antibacterial agent are as described above. The method for administering the antibacterial agent may be, for example, oral administration or vaginal administration. From the viewpoint of reducing the effect of the antibacterial agent on other parts of the body, the administration method is preferably vaginal administration. The dosage of the antibacterial agent is not particularly limited as long as it is a therapeutically effective amount, and may be determined depending on the type of the antibacterial agent.

### Examples

### <Materials>

Endometrial and ovarian tissues used in test examples below were collected from patients at Nagoya University Hospital and its affiliated hospitals. All mice used in the test examples below are female BALB/c mice (Japan SLC, Inc.). *Fusobacterium nucleatum* and *Lactobacillus iners* (indigenous bacterium in the vagina) with which the mice were infected were obtained from the Center for Conservation of Microbial Genetic Resource, Organization for Research and Community Development, Gifu University. *F. nucleatum ssp. nucleatum* (strain ATCC25586) used in Test Example 5 was obtained from Riken, a National Research and Development Agency. 17β-estradiol administered to mice was manufactured by Fuji Pharma Co., Ltd. Metronidazole (MZ) administered to mice was manufactured by FUJIFILM Wako Pure Chemical Corporation, and chloramphenicol (CP) was manufactured by Sigma Corporation.

### <FISH>

In the following test examples, analysis by fluorescence in situ hybridization (FISH) was performed as follows. First, slides of formalin-fixed paraffin-embedded tissue slices were deparaffinized and treated with 0.2 M HCl for 20 minutes, and then hybridized with 10 ng/µL FISH probes in a hybridization buffer solution (0.9 M NaCl, 20 mM Tris-HCl, pH 7.2, 0.1% sodium dodecyl sulfate) at 56°C overnight. As the FISH probes, 5'-FAM-labeled eubacterial universal probe EUB338, 5'-Cy3-labeled *Fusobacterium* bacteria-specific probe FUSO, and 5'-Cy5-labeled *F. nucleatum*-target probe FUS664 were used. FISH probe sequences were obtained from probeBase (http://probebase.csb.univie.ac.at/) (accession number: pB-159, pB-782, and pB-1346). The slides were washed with a wash buffer solution (0.9 M NaCl, 20 mM Tris-HCl, pH 7.2) at 58°C for 20 minutes. The tissue slices were counterstained with 4',6-diamidino-2-phenylindole (DAPI) and mounted on glass slides. Opal Multi-plex IHC Detection Kit (manufactured by PerkinElmer, Inc.) was used to perform co-immunostaining with an anti-TAGLN antibody and the FISH probes according to the manufacturer's protocol. Fluorescence images were acquired using an inverted microscope Leica DMI6000B (manufactured by Leica Microsystems).

### <Test Example 1> Detection of a Fusobacterium bacterium in endometrial tissue

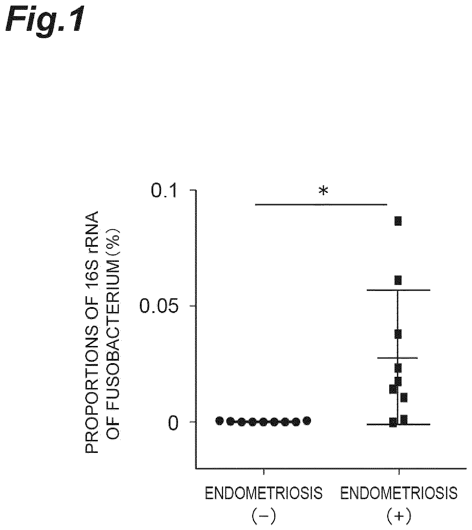

QIAamp DNA Microbiome Kit (manufactured by QIAGEN) was used to extract genomic DNAs from normal human endometrial tissues (n=9) and endometrial tissues (n=9) from patients with endometriosis. A quantitative polymerase chain reaction (qPCR) was performed using primers specific *Fusobacterium* bacteria, and the proportions of 16S rRNA of *Fusobacterium* within the total 16S rRNA were calculated by a ΔCT method. The results are shown in FIG. 1. 16S rRNA of *Fusobacterium* was not detected in the normal human endometrial tissues, but was detected in the endometrial tissues from the patients with endometriosis (*P<0.05).

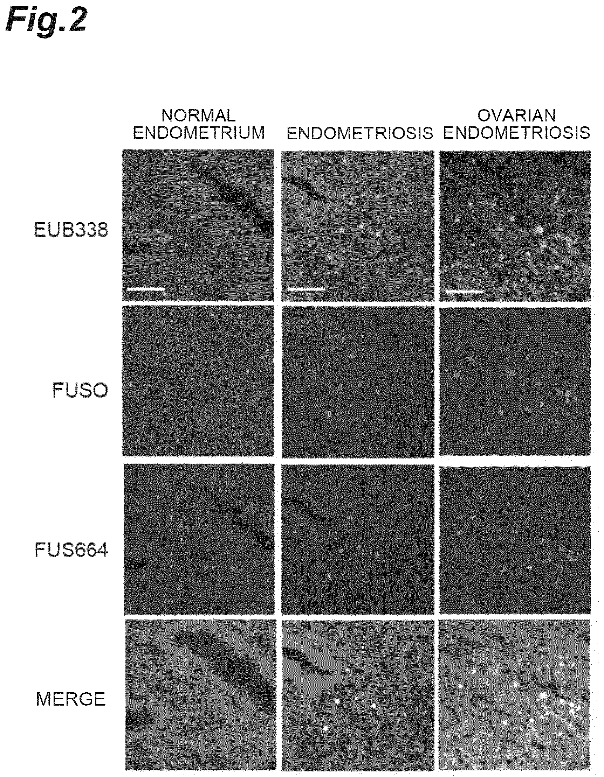

Next, the normal human endometrial tissues, and the endometrial tissues and ovarian tissues from the patients with endometriosis were subjected to FISH using EUB338 specific to eubacteria, FUSO specific to *Fusobacterium* bacteria, and FUS664 specific to *F. nucleatum* as probes. Fluorescence images are shown in FIG. 2 (with a scale bar being 100 µm). Fluorescence of EUB338, FUSO, and FUS664 was observed in the endometrial tissues and ovarian tissues from the patients with endometriosis, indicating that these tissues were infected with *F. nucleatum.*

Next, the positive rates of the *Fusobacterium* bacteria in the endometrial tissues from the patients without endometriosis and the patients with endometriosis were investigated by FISH. The fluorescence of FUSO was observed in 3 of 42 patients who were not suffering from endometriosis (positive rate: 7%) and in 27 of 42 patients with endometriosis (positive rate: 64%).

### <Test Example 2> Induction of endometriosis in mice

The ovaries of 6-week-old donor mice were removed, and 10⁷ CFU (colony-forming units)/10 µL PBS/day of *F nucleatum* or *L. iners* were intravaginally applied 4 times a week to infect the donor mice with the bacteria. Phosphate-buffered saline (PBS) was applied to a control group. Two weeks after the removal of the ovaries, endometrial tissues collected from the donor mice were inoculated intraperitoneally into recipient mice to induce endometriosis. Subcutaneous administration of 17β-estradiol at 100 µg/kg/week was started after the ovariectomy for the donor mice and after the endometrial tissue inoculation for the recipient mice. Four weeks after the intraperitoneal inoculation, peritoneal endometriotic lesions of the recipient mice were excised, and the number and total weight of the lesions were measured.

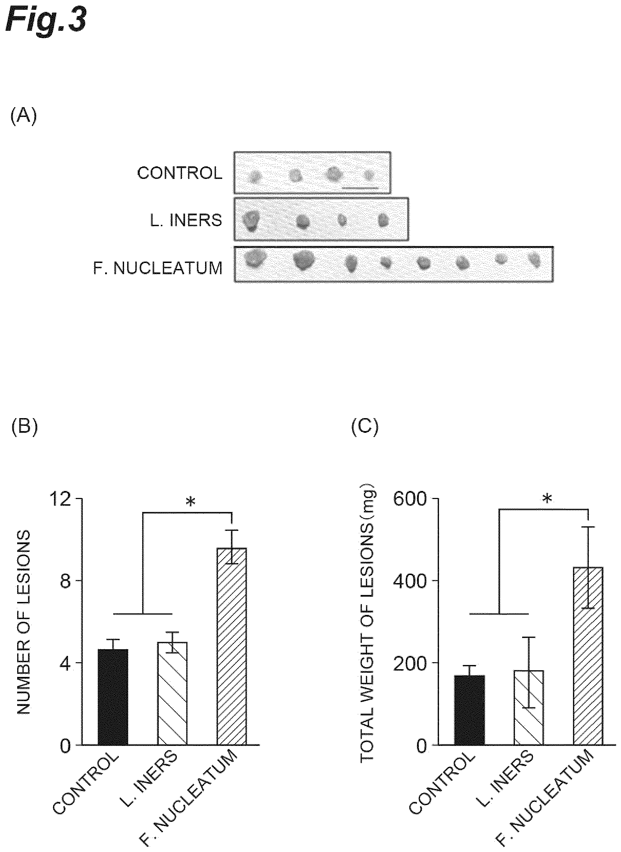

FIG. 3(A) shows the excised lesions. FIG. 3(B) shows the number of the lesions, and FIG. 3(C) shows the total weight of the lesions (*P<0.05, n=3 for each group). As shown in these drawings, the number and total weight of the lesions were significantly high in the recipient mice inoculated with the endometrial tissues infected with *F*. *nucleatum.*

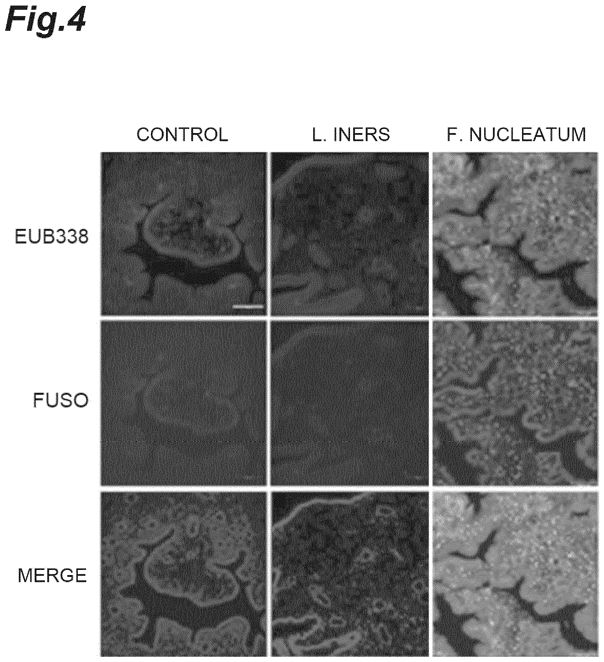

FISH was performed on the lesions using EUB338 and FUSO as probes. Fluorescence images are shown in FIG. 4 (with a scale bar being 100 µm). *Fusobacterium* bacteria were detected in the endometriotic lesions of the recipient mice inoculated with the endometrial tissues infected with *F. nucleatum.*

### <Test Example 3> Administration of antibacterial agent to donor mice

The ovaries of 6-week-old donor mice were removed, and 10⁷ CFU /10 µL PBS/day of *F nucleatum* were intravaginally applied 4 times a week to infect the donor mice with *F nucleatum.* One week after the removal of the ovaries, an antibacterial agent containing 17 µg/day metronidazole (MZ) or 7 µg/day chloramphenicol (CP) was administered vaginally daily for 5 days. PBS was administered to the control group. Three weeks after the removal of the ovaries, endometrial tissues collected from the donor mice were inoculated intraperitoneally into recipient mice to induce endometriosis. Subcutaneous administration of 17β-estradiol at 100 µg/kg/week was started after the ovariectomy for the donor mice and after the endometrial tissue inoculation for the recipient mice. Three weeks after the intraperitoneal inoculation, peritoneal endometriotic lesions of the recipient mice were excised, and the number and total weight of the lesions were measured.

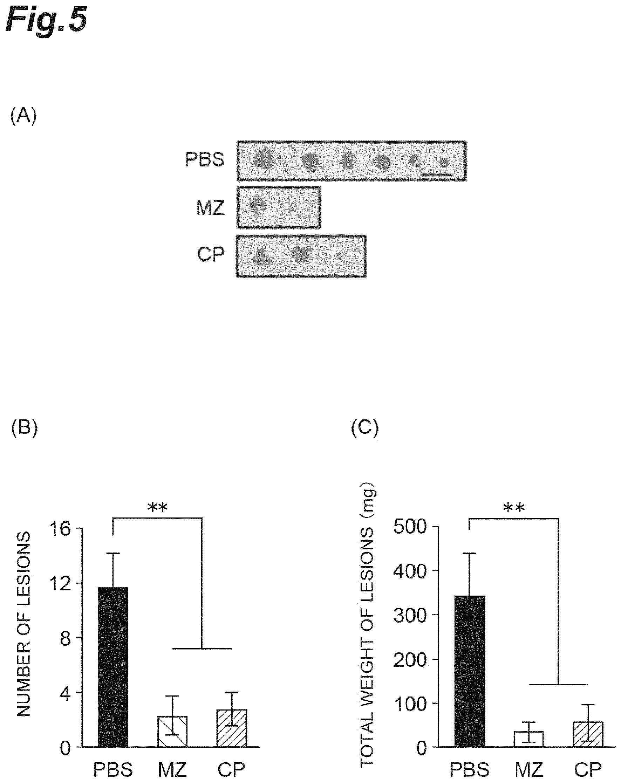

FIG. 5(A) shows the excised lesions. FIG. 5(B) shows the number of the lesions, and FIG. 5(C) shows the total weight of the lesions (**P<0.01, n=3 for each group). As shown in these drawings, the number and total weight of the lesions were significantly reduced in the recipient mice inoculated with the endometrial tissues from the donor mice treated with the antibacterial agent.

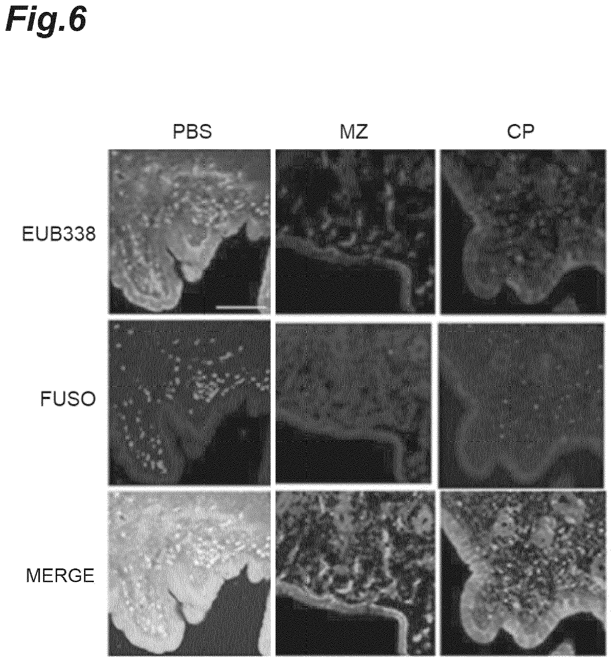

FISH was performed on the lesions using EUB338 and FUSO as probes. Fluorescence images are shown in FIG. 6 (with a scale bar being 100 µm). *Fusobacterium* bacteria were detected in the control group inoculated with the endometrial tissues of the donor mice to which no antibacterial agent was administered, whereas no *Fusobacterium* bacteria were detected in the recipient mice inoculated with the endometrial tissues from the donor mice treated with the antibacterial agent.

### <Test Example 4> Administration of an antibacterial agent to recipient mice

The ovaries of 6-week-old donor mice were removed, and 10⁷ CFU/10 µL PBS/day of *F nucleatum* were intravaginally applied 4 times a week to infect the donor mice with *F nucleatum.* Two weeks after the removal of the ovaries, the endometrial tissues collected from the donor mice were inoculated intraperitoneally into recipient mice to induce endometriosis. Subcutaneous administration of 17β-estradiol at 100 µg/kg/week was started after the ovariectomy for the donor mice and after the endometrial tissue inoculation for the recipient mice. Three weeks after the intraperitoneal inoculation, an antibacterial agent containing 17 µg/day metronidazole or 7 µg/day chloramphenicol was administered to the recipient mice orally daily for 5 days. 21 days after the end of administration, peritoneal endometriotic lesions of the recipient mice were excised, and the number and total weight of the lesions were measured.

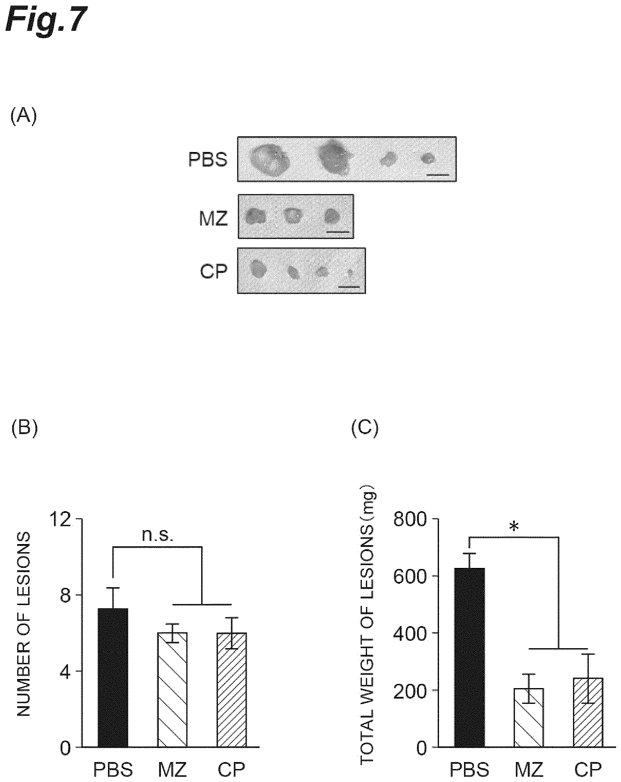

FIG. 7(A) shows the excised lesions (with a scale bar being 1 cm). FIG. 7(B) shows the number of the lesions, and FIG. 7(C) shows the total weight of the lesions (*P<0.05, n.s.: no significant difference, n=3 for each group). As shown in these drawings, for the endometriosis mice treated with the antibacterial agent, the total weight of the lesions was significantly reduced, although there was no significant change in the number of the lesions.

When FISH was performed on the lesions using EUB338 and FUSO as probes, the amount of *Fusobacterium* bacteria was dramatically reduced in the endometriosis mice treated with the antibacterial agent (not shown in the drawings).

### <Test Example 5> Detection of a Fusobacterium bacterium in menstrual blood

10 µL of a bacterium liquid of *F nucleatum ssp. nucleatum* (strain ATCC25586) was added to 490 µL of menstrual blood negative for *Fusobacterium* bacteria, and DNA was extracted from 500 µL of the obtained menstrual blood sample using QIAamp DNA Microbiome Kit. A LAMP reaction was performed on the obtained DNA using a *F. nucleatum ssp. nucleatum*-specific primer set shown in Table 1. The reaction was performed at 65°C for 120 minutes. Detection of an amplification product was performed by monitoring the fluorescence intensity of SYTO (trademark) 63 Red Fluorescent Nucleic Acid Stain (manufactured by Thermo Fisher Scientific Inc.). The time required for a moving average value of the fluorescence intensity to reach a threshold value a certain number of times was measured, and the first time the threshold value was exceeded was defined as a Tt value. As a control, 10 µL of physiological saline was added to 490 µL of menstrual blood negative for *Fusobacterium* bacteria, and using the obtained menstrual blood sample, DNA extraction and LAMP reaction were performed in the same manner as described above. The Tt values are shown in Table 2.

**[Table 1]**

| Primer | SEQ ID NO. | Sequence (5' to 3') |
|---|---|---|
| F3 | 1 | TAGGAAATACTTGTAATATGAATGGT |
| B3 | 2 | GTATATCCGATATCAGTTATAGGC |
| FIP | 3 | CGAAAGAACTAATCCCATAAATACAAATTG-GTTGGATTAGG TGTAATAAGTGT |
| BIP | 4 | GGATGTGCTGGGGTAAAAGG-AGCTGCTAAAATTGGTATCAG TG |
| LF | 5 | AGTAATGATACAAAAGTTATTGGATAACCATAAATATTTGAA GC |
| LB | 6 | GTCAACAGTTTTATTACAAACATTTGGAATGCC |

**[Table 2]**

| | | | |
|---|---|---|---|
| Amount of bacterium of *F. nucleatum* (CFU) | 2×10⁸ | 2×10⁴ | No bacterium |
| Tt value (minute) | 11.7 | 22.8 | Not detected |

No amplification product of the LAMP reaction was detected in the menstrual blood sample containing no *F. nucleatum ssp. nucleatum* (control), whereas an amplification product was detected in the menstrual blood sample containing *F nucleatum ssp. nucleatum.* Therefore, it was shown that when a *Fusobacterium* bacterium derived from an endometrium is present in menstrual blood, the bacterium can be detected by a nucleic acid amplification method.

## Claims

1. A method for detecting a *Fusobacterium* bacterium for diagnosing endometriosis, comprising:
detecting a *Fusobacterium* bacterium in a sample collected from a subject,
wherein a detection of a presence of a *Fusobacterium* bacterium in the sample indicates that the subject is likely suffering from or likely to suffer from endometriosis.

2. The method according to claim 1, wherein the *Fusobacterium* bacterium is *Fusobacterium nucleatum.*

3. The method according to claim 1 or 2, wherein the sample is blood, plasma, serum, urine, endometrial cells, vaginal swab fluid, or menstrual blood.

4. A therapeutic agent for endometriosis comprising:
an antibacterial agent against a *Fusobacterium* bacterium as an active component.

5. A method for treating endometriosis, comprising:
a step of administering an antibacterial agent to a subject whose endometrium is infected with a *Fusobacterium* bacterium.

6. The method according to claim 5, further comprising:
a step of detecting a *Fusobacterium* bacterium in a sample collected from the subject to confirm whether or not the endometrium of the subject is infected with the *Fusobacterium* bacterium before the step of administering an antibacterial agent.

7. The method according to claim 5 or 6, wherein the antibacterial agent is administered vaginally.
